# EUROPEAN PATENT SPECIFICATION

(11) **EP 1 670 528 B1**
(45) Date of publication and mention of the grant of the patent: **17.06.2015**
(21) Application number: 04784632.4
(22) Date of filing: 21.09.2004
(51) Int. Cl.: A61M 5/32, A61M 5/50

(54) **SAFETY NEEDLE ASSEMBLY AND METHOD FOR MAKING THE SAME**
SICHERHEITSNADELANORDNUNG UND HERSTELLUNGSVERFAHREN DAFÜR
DISPOSITIF D'AIGUILLE DE SECURITE ET SON PROCEDE DE CONCEPTION

(30) Priority: 22.09.2003 US 665514; 27.04.2004 US 832339
(43) Date of publication of application: 21.06.2006
(73) Proprietor: Smiths Medical ASD, Inc., Keene, NH 03431-0724 (US)
(72) Inventor: HAURI, Marius, Westmoreland, NH 03467 (US); BLINKHORN, Frank, Keene, NH 03431 (US); MACLEAN, David, Swanzey, NH 03446 (US); HUDON, Lawrence, P., Hinsdale, NH 03451 (US); SIMAS, Robert, Jr., Keene, NH 03431 (US); DERBY, Troy, M., Stoddard, NH 03464 (US)
(74) Representative: Bennett, Adrian Robert J.
(86) International application number: PCT/US2004/030830
(87) International publication number: WO 2005/030290

(56) References cited:
- EP-A1- 1 186 313
- US-A- 5 490 841
- US-A- 5 643 219
- US-A- 5 669 889
- US-A- 5 681 295

## Description

The present invention relates to needles and more particularly a safety needle assembly in which the needle sheath for protecting the needle prior to use is attached to a collar rotatably mounted about the needle hub of the needle assembly.

There are a number of needle protection devices disclosed in the prior art. Among them are a number of patents assigned to the same assignee as the instant invention. Without limitations, some of those patents are: 4,982,842; 5,139,489; 5,154,285; 5,232,454; 5,277,311; 5,993,426; 6,328,713; 6,334,857; RE37,110 and RE37,252. Some other patents that describe needle protection devices, or parts thereof, include U.S. patents: 4,664,259; 5,037,401; 5,171,303; 5,188,611; 5,490,841; 5,509,907; 5,584,816; 5,599,313; 5,599,318; 5,632,732; 5,643,219; 5,662,617; 5,665,075; 5,669,889; 5,681,295; 5,697,908; 5,733,265; 5,868,716; 5,891,103; 5,913,846; 5,919,165 and 6,440,104.

The needle protection assembly of the instant invention is made up of parts that are radically different from the prior art, as exemplified by the above-noted patents.

US 5643219, upon which the precharacterising portion of claim 1 is based, disposes Safety apparatus, comprising: a needle hub having a proximal portion and a distal portion, a needle extending from a distal end of said needle hub; a collar rotatably mounted about the distal portion of said needle hub; a housing pivotally connected to said collar; and a needle sheath attached to said collar for covering said needle extending from the distal end of said needle hub.

Another prior art arrangement is disclosed in EP 1186313.

According to the first aspect of the present invention there is provided a safety apparatus as defined in claim 1.

The present invention further defines, in combination with a needle hub and a collar assembly as defined in claim 11.

The present invention still further provides a method of making a needle assembly as defined in claim 20.

The safety needle assembly of the instant invention is designed to enable a user to connect the needle hub to a medical device, such as for example a syringe, by grasping the needle hub proper, thereby ensuring a more secure fit to the syringe. Unlike the prior art needle assemblies, the sheath that covers the needle plays no part in the securing of the needle hub to the medical device.

The collar to which a needle protection housing is preferably attached is fitted to the space defined by the flanges and the distal wall of the ring on the needle hub. To facilitate the fitting, a number of internal protrusions or bosses are advantageously provided at the proximal end of the collar. The respective surfaces of the protrusions that come into contact with the flanges at the needle hub are also chamfered to facilitate the mating of the collar to the needle hub. The back end of the substantially rectangular protrusions are flat, so that once the collar is fitted to the needle hub, it could not be removed therefrom.

At the distal portion of the collar there is formed a circumferential internal rib. Slots are also provided at the distal portion of the collar to enable the flexing of the distal end of the collar for the insertion and removal of a needle sheath that removably couples thereto.

The collar has preferably pivotally or hingedly attached thereto a housing which is pivotable to the direction along a longitudinal axis of the needle hub for covering the needle after use. Preferably, formed substantially along the length of the housing is an opening that is off centered. The opening is formed by two lips or flaps that extend substantially along the length of the housing, with the first or upper lip overlapping the second or lower lip. The respective lips each are angled toward the interior of the housing, but with varying angles along the lengths of the lips. As a consequence, when the housing is pivoted to cover a used or contaminated needle, the needle would enter into the housing guided by the lips at angles that ensure that it smoothly enters into the housing, thereby preventing flickering of any contaminated fluid that may have adhered to the needle. The lips, particularly the lower lip, are designated such that, once fully enters into the housing, the needle is prevented from escaping from the housing. For added safety, respective portions of a locking mechanism are preferably provided at the base portion of the housing and the outer surface of the distal portion of the collar.

The needle sheath that covers the needle prior to use preferably has a notch or groove formed circumferentially proximate to its open end. During manufacturing of the needle assembly, the needle sheath is placed or positioned over the needle and moved along the longitudinal axis of the device to mate with the distal portion of the collar mounted about the needle hub. With a predetermined force, the needle sheath is coupled to the collar, with the rib at the distal end of the collar fitting into the groove formed at the proximal end of the needle sheath.

To remove the safety needle assembly of the instant invention from the medical device, the user would grasp the ring of the needle hub and rotate the needle assembly in a rotational movement that is counter to the rotational movement used to couple the needle assembly to the medical device, if the coupling of the needle assembly to the medical device is via luer lock coupling. If it is a luer fit coupling, then the user would pull the needle assembly away from the medical device.

Other embodiments of the safety needle assembly of the instant invention enable a user to connect the needle hub to a medical device with or without grasping the needle hub proper.

A first alternative embodiment of the safety needle assembly of the instant invention has a needle sheath that has a base that is larger in diameter than the collar, so that the needle sheath may be slip fitted over the collar. The base of the needle sheath is provided with two opposed slots or keyways that, when the needle sheath is slip fitted over the collar, each of the slots would mate with a corresponding catch member formed at the outer wall of the collar. As a consequence, the collar is rotatable in unison with the needle sheath, when the needle sheath is rotated by a user. For this alternative embodiment, the needle hub is the same as in the earlier embodiment so that to connect and disconnect the needle assembly from the luer end of a syringe, a user would still need to grasp the needle hub.

Another alternative embodiment of the needle assembly of the instant invention has a needle sheath that has a proximal open end to which opposed keys or protuberances are provided to mate with keyways or slots formed at the distal portion of the collar. Thus, once the needle sheath is fitted to the collar, the collar is rotatable in unison with the needle sheath, when the user rotates the needle sheath. The collar of this embodiment also has a proximal portion at the end of which there is a flange. The needle hub of this embodiment is provided with a ring at the portion to which the collar is rotatably mounted. The ring has two end stops, along with at least one protuberance along the edge between the stops, for providing guidance to the rotational positioning of the collar relative to the needle hub. Once the collar is rotatably mounted about the needle hub, the flange at its proximal end would coact against the edge of the ring between the two end stops, so that the collar could be rotated between the two end stops 180°, with the protuberance possibly providing an indication for the user that the collar is at a midpoint between the two end stops relative to the needle hub. The midpoint positioning of the collar relative to the needle hub allows a user to align the bevel end of the needle away from the housing, so that the user could have a clear view of the bevel of the needle for insertion into the vein of a patient. With the end stops, the needle assembly of this embodiment may be connected to and disconnected from a syringe, respectively, by the user turning the needle sheath, prior to the needle being exposed, and turning the housing, after the needle sheath has been removed and after the housing has been pivoted to cover the contaminated needle.

Another embodiment of the safety needle assembly of the instant invention has the just mentioned needle sheath. But for this embodiment, the collar is designed to have a number of fingers extending at its proximal end and a number of catches or heads that extend from the internal pads of the collar that allow the collar to be rotatable about the needle hub. As before, the needle hub has two sets of orthogonal flanges extending from its body for defining a space or groove about which the pads of the collar are movably mounted. To connect the needle assembly of this embodiment to a syringe, a user would hold the needle sheath and push it forward toward the syringe so that some of the fingers extending from the proximal end of the collar come into contact with the aft set of flanges, or rather the edges thereof, of the needle hub, so that the needle hub is rotated, when the needle sheath is rotated, for mating the needle hub to the luer end of the syringe. To remove the needle assembly after use, assuming that the needle sheath has been removed and the contaminated needle is covered by the needle housing which had been pivoted to cover the same and lockingly retained to the collar, a user would pull the collar away from the syringe so that some of the heads of the internal pads of the collar come into contact with the fore flanges, or rather the edges of those flanges, so as to enable to the user to rotatably remove the needle hub from the syringe.

Yet another embodiment of the safety needle assembly of the instant invention has a needle sheath that has an enlarged proximal end dimensioned to slip fit over the needle collar. The enlarged proximal end of the needle sheath is designed to have a slot that mates with the portion of the living hinge that extends from the collar for connecting the needle protection housing. A pair of opposed spring arms are provided at the proximal end of the housing, so that when the needle sheath is slip fitted over the collar, those spring arms are pushed inwardly toward the interior of the collar, which is rotatably mounted about a portion of the needle hub defined by two sets of spaced flanges. The space or groove on the needle hub of this embodiment of the needle assembly has two flat sections that enable the spring arms to press against the needle hub in a rest position, when the needle sheath is fitted over the collar. Thus, before the needle sheath is removed from the collar, a user can readily connect the needle hub, which rotates in unison with the collar, which in turn rotates in unison with the needle sheath, to the luer end of a syringe. The housing for this alternative embodiment of the needle assembly has a pair of legs or protrusions extending at its open end so that, when the housing is pivoted to cover a contaminated needle, the protrusions would bias against the spring arms at the collar to thereby press those spring arms against the flat sections of the needle hub about which the collar is mounted. As a consequence, by rotating the housing, the needle hub likewise is rotated and therefore is removable from a syringe.

Yet another embodiment of the needle assembly of the instant invention is designed to include a collar that has a proximal portion that rotatably mounts about a needle hub and a number of internal spring fingers that extend from the proximal portion of the collar to the distal portion of the collar. The needle hub of this embodiment of the needle assembly is designed to be similar to that of the incorporated by reference '837 application in that it has a number of arms formed coplanarly at the distal portion of the needle hub. Once the collar is rotatably mounted about the proximal portion of the needle hub, with the insertion of the needle sheath into the collar, the internal spring arms of the collar are biased inwards to rest within spaces defined by adjacent pairs of the coplanar arms. As a consequence, the needle hub rotates in unison with the collar and the needle sheath, which is designed to press-fit into the collar, is able to rotate the collar by its tight fit thereto. The needle assembly of this embodiment of the instant invention can therefore be readily connected to a syringe. The housing of this embodiment of the needle assembly is designed to have a leg extending from its open end to bias one of the spring arms inward, when the housing is pivoted to cover the exposed needle. With the spring arm biased inward to be positioned between a space defined by adjacent coplanar arms at the distal portion of the needle hub, a user can rotate the housing to cause the needle hub to likewise rotate, thereby removing the needle hub from the syringe.

In order that the invention may be well understood, there will now be described an embodiment thereof given by way of example, reference being made to the accompanying drawings, in which:
Fig. 1 is a perspective view showing the different component parts of the safety needle assembly of the instant invention;
Fig. 2 is a perspective view showing the various components of the safety needle assembly of Fig. 1 and a medical device such as a conventional needle syringe to which the safety needle assembly of the instant invention is used with;
Fig. 3 is a perspective view of the safety needle assembly of the instant invention with all of the components assembled;
Fig. 4 is a cross-sectional view of the safety needle assembly of the instant invention;
Fig. 5 is a perspective cross-sectional view of the needle assembly of the instant invention;
Fig. 6 is a perspective view of the needle hub of the safety needle assembly of the instant invention as viewed from its proximal end;
Fig. 7 is a perspective view of the needle hub of the instant invention safety needle assembly viewed from its distal end;
Fig. 8 is a perspective view of the needle protection housing and the collar to which it is attached;
Fig. 9 is another perspective view of the Fig. 8 needle protection housing and collar;
Fig. 10 is yet another view of the needle protection housing and collar of the instant invention safety needle assembly, with the lips that form the longitudinal slot along the housing clearly shown;
Fig. 11 is a plan view of the needle protection housing and the collar component of the safety needle assembly of the instant invention;
Fig. 12 is a perspective view of the needle sheath of the instant invention safety needle assembly as viewed from its open end;
Fig. 13 is another perspective view of the needle sheath of Fig. 12 but viewed from its closed end;
Fig. 14 is a perspective view of an alternative embodiment of the needle assembly of the instant invention;
Fig. 15 is another alternative embodiment of the needle assembly of the instant invention;
Fig. 16 is yet another alternative embodiment of the needle assembly of the instant invention;
Fig. 17 is still yet another alternative embodiment of the needle assembly of the instant invention; and
Fig. 18 is a cross-sectional view of still yet another alternative embodiment of the needle assembly of the instant invention.

### Detailed Description of the Invention

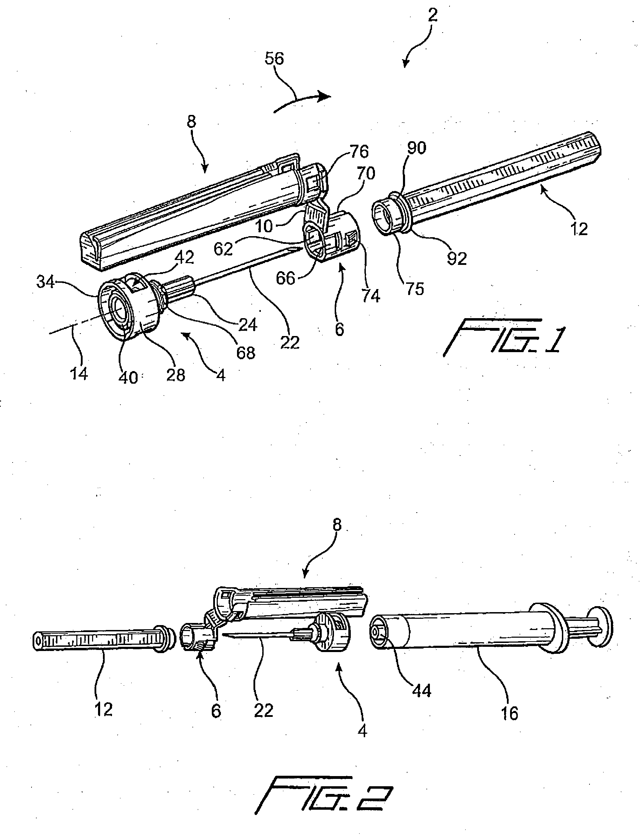

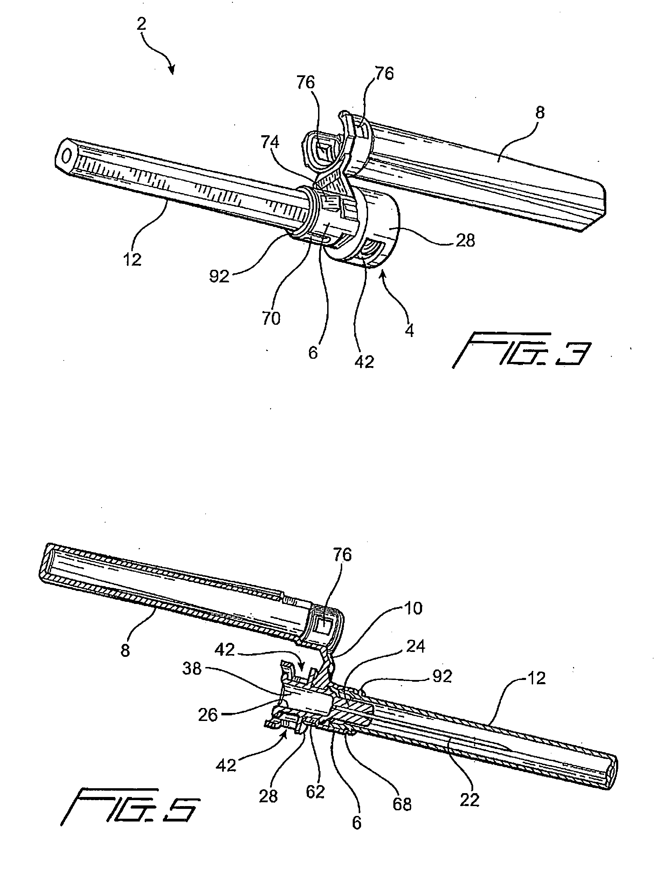

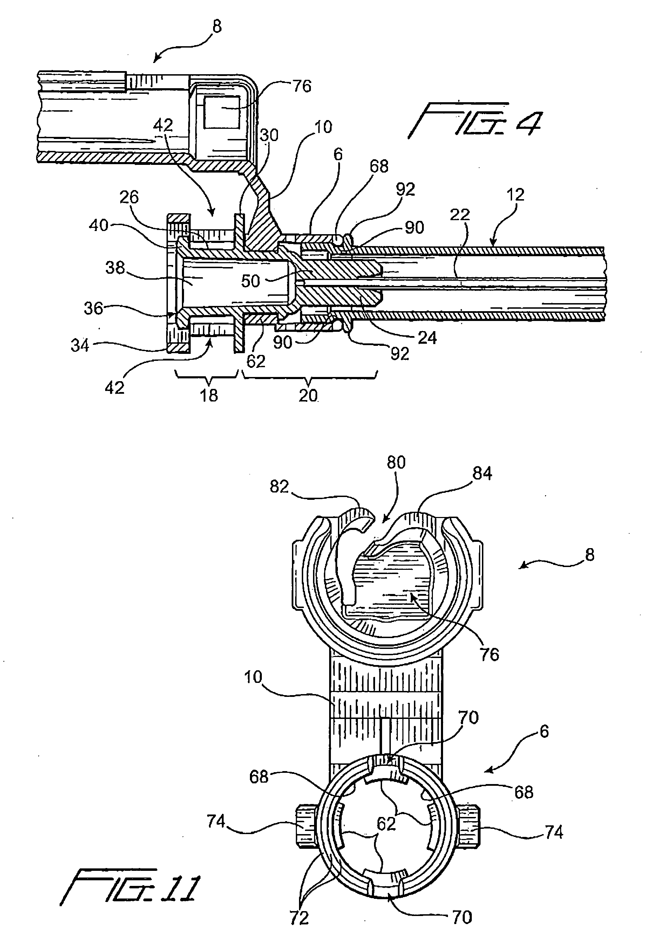

With reference to Fig. 1, the safety needle assembly 2 of the instant invention is shown to comprise four major components, namely a needle hub 4, a collar 6, a needle protection housing 8 attached to the collar 6 via a living hinge 10, and a needle sheath 12. As shown, needle hub 4, collar 6 and needle sheath 12 are in alignment along a longitudinal axis 14. The exposed components of the safety needle assembly of the instant invention are further shown in Fig. 2 to be in alignment with a conventional syringe 16 with a luer lock receptacle end that mates with needle hub 4. An assembled safety needle, assembly of the instant invention is shown in perspective view in Fig. 3 and in cross-sectional views in Figs. 4 and 5.

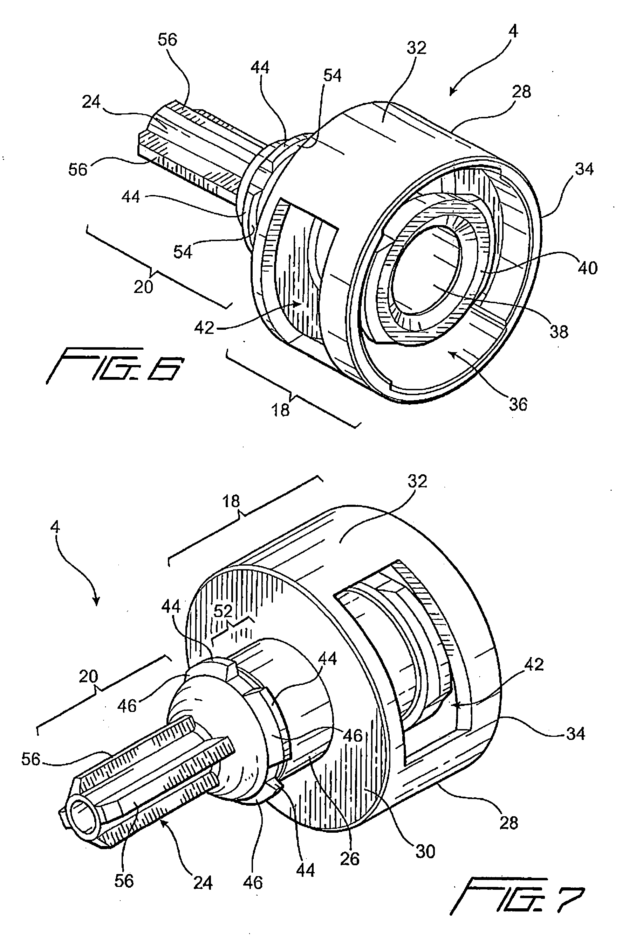

With reference to Figs. 1-5 and further with reference to Figs. 6 and 7, needle hub 4 is shown to have a proximal portion 18 and a distal portion 20. For the sake of clarity, a needle 22 that fixedly extends from extension 24 of needle hub 4 is not shown in Figs. 6 and 7. Further, it should be appreciated that proximal portion 18 and distal portion 20 of needle hub 4 do not have any actual line of demarcation, and are shown as such in Figs. 4, 6 and 7 solely for the convenience of the reader.

As best shown in Fig. 6 and the cross-sectional views of Figs. 4 and 5, needle hub 4 comprises a main body portion 26 that includes the base of needle hub 4. A ring 28 circumferentially surrounds the proximal portion of needle hub 4 in spaced relationship to main body portion 26. As shown, ring 28 is a part of needle hub 4 and is an integral part of main body portion 26 by means of a distal wall or partition 30 that extends transversely or orthogonally from main body portion 26. From distal wall 30 the sidewall 32 of collar 28 extends to a proximal end 34 that has an opening 36 formed concentrically with opening or cavity 38 at luer end 40 of needle hub main body portion 26. Since sidewall 32 of ring 28 is in spaced relationship with main body portion or base 26 of needle hub 4, luer end 40 of needle hub 4 accordingly is threadingly matable with a corresponding luer connector such as that shown for syringe 16 in Fig. 2.

Ring 28 is also provided with two openings or windows 42 along its sidewall 32 to enable a user to view base portion 26 of needle hub 4. As needle hub 4, and the other components of the needle assembly, are made of conventional medical plastic such as polypropylene, or ABS plastic, and is substantially clear except for a color tinting as a way of color coding the assembly, the user can readily ascertain any flashing or blood, or blood flash, during a blood withdrawing procedure to thereby determine whether needle 22 has been correctly inserted into the vein of a patient. Thus, by way of windows 42, a user can view the base portion, as well as luer end 40 of needle hub 4. It should of course be appreciated that the safety needle assembly of the invention can also be used for infiusion procedure. The dimension of ring 28 is such that it enables the user to readily grasp needle hub 4, and therefore the safety needle assembly as shown in Fig. 3, for mating to syringe 16 as shown in Fig. 2. As is well known, luer end 40 of needle hub 4 may be coupled to the corresponding luer end 44 of syringe 16 by a rotational movement when syringe 16 has a luer lock type receptacle, as shown in Fig. 2. Alternatively, in the case where the syringe has a luer slip type receptaele, the user, upon grasping ring 28, can simply insert luer 40 onto the luer slip receptacle of the syringe.

Further with respect to Figs. 1-7 and in particular with respect to Figs. 6 and 7, at the distal portion 20 of needle hub 4 there are provided a number of flanges 44 in a coaxially circumferential manner a predetermined distance from end wall 30 of ring 28. Flanges 44 each are chamfered or beveled at its front surface 46, and extend circumferentially proximate to the front end of cavity 38 of the base portion 26 of needle hub 4. As best shown in Figs. 4 and 5, cavity 38 of base portion 26 is connected to needle 22 by a through bore 50 at extension 24. Extension 24 has a number of elongated ribs 56, which has no bearing for this invention other than for cosmetic and manufacturing processes not related to the instant invention, as ribs 56 do not come into contact with needle sheath 12 at any time. With flanges 44 extending from base portion 26 a predetermined distance from end wall 30, a space 52 is defined between flanges 44 and end wall 30 circumferentially about base portion 26. As best shown in Fig. 6, the back surfaces 54 of flanges 44 are formed at right angle to base portion 26.

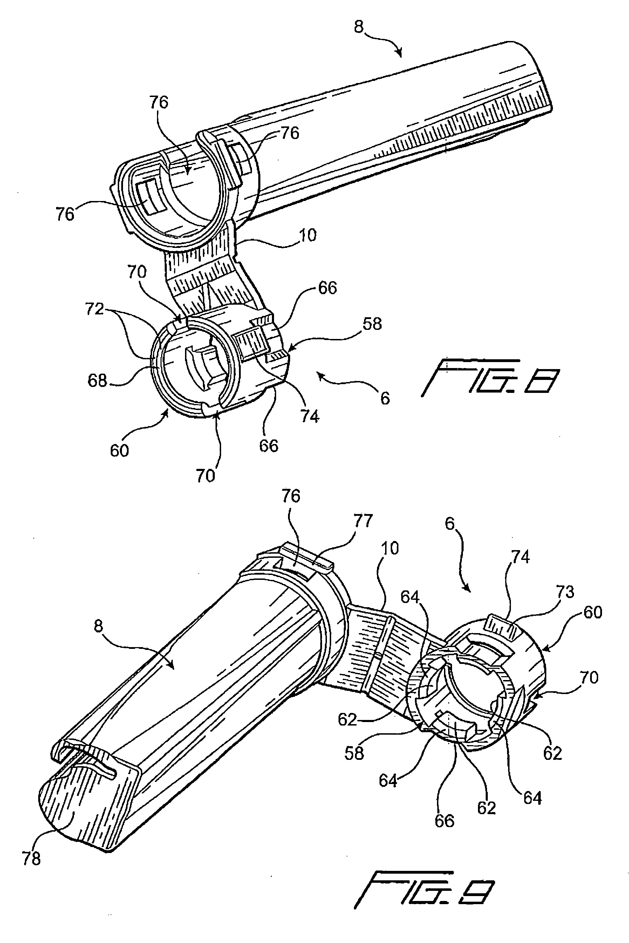

As shown in Figs. 1-5, needle hub 4 is in alignment with collar 6 and is coupled thereto per shown in the assembled views of Figs. 3-5. In particular, with reference to Figs. 8-11, collar 6 is pivotally connected to needle protection housing 8 by living hinge 10. As shown in Fig. 1, housing 8 is pivotable in the direction indicated by directional arrow 56, i.e., toward the longitudinal axis 14 for covering needle 22.

Collar 6 is cylindrical in shape and has a proximal portion or end 58 and a distal portion or end 60. There are formed at the inner surface at proximal portion 58 of collar 6 a plurality of protrusions 62 which are substantially rectangularly shaped. Protrusions 62 each may have a chamfered surface 64 that faces needle hub 4, as shown in the alignment of the components illustration of Fig. 1. Moreover, protrusion 62 are dimensioned such that when collar 6 is press-fitted to needle hub 4, they will matingly fit to space 52 defined by flanges 54 and end wall 30 at the distal portion of needle 4. The respective dimensions of space 52 and protrusions 62 may be such that, although collar 6 is rotatable about base portion 26 of needle hub 4, there nonetheless is enough friction between either one of flanges 44, end wall 30 or the outer surface of needle hub base 26 and protrusions 62 to render collar 6 not freely rotatable about needle 4, unless a predetermined torque or force is applied either to needle protection housing 8, living hinge 10 or collar 6, to rotate collar 6 relative to needle hub 4. Voids 66 provided at proximal portion 58 of collar 6 enable proximal portion 58 to flex, or expand, when collar 6 is press-fitted to the distal portion of needle hub 4, particularly when protrusions 62 come into contact with flanges 44. The respective chamfered or beveled surfaces 46 and 64 of flanges 44 and protrusions 62, respectively, facilitate the insertion of collar 6, and more particularly protrusions 62 into space 52 of needle hub 4.

Distal portion 60 of collar 6 has at its distal end, or proximate thereto, a rib 68 formed at the inner surface of collar 6. For the embodiment shown, rib 68 is divided into two halves, per notches 70 formed at opposed sides of distal portion 60. Notches 70 provide additional flexibility to the distal portion of collar 6 when needle sheath 12 is fitted thereto. More on that later. For now, it should be appreciated that rib 68 is formed to have either a semi-circular configuration or a configuration that is made up of a number of beveled surfaces for facilitating the mating of distal portion 60 of collar 6 with the proximal portion 75 of needle sheath 12. The beveled surfaces of rib 68 are collectively designated 72.

Needle protection housing 8 is connected, by living hinge 10, to collar 6 at the latter's proximal portion 58. Needle protective housing 8 has an open proximal end 75 and a closed end 78. Housing 8 is cylindrical in shape and has an opening 80 through which needle 22 passes, when housing 8 is pivoted toward collar 6 for covering needle 22 after needle sheath 12 has been removed from collar 6. Opening or channel 80 is formed by two lips or flaps 82 and 84 each of which extends longitudinally along the entire length of housing 8. Lip 82 overlaps lip 84, with the overlapping being such that the combination of lips 82 and 84 providing a trap door for needle 22. Thus, once needle 22 passes lips 82 and 84 into housing 8, it is trapped within housing 8 and is prevented from being further exposed.

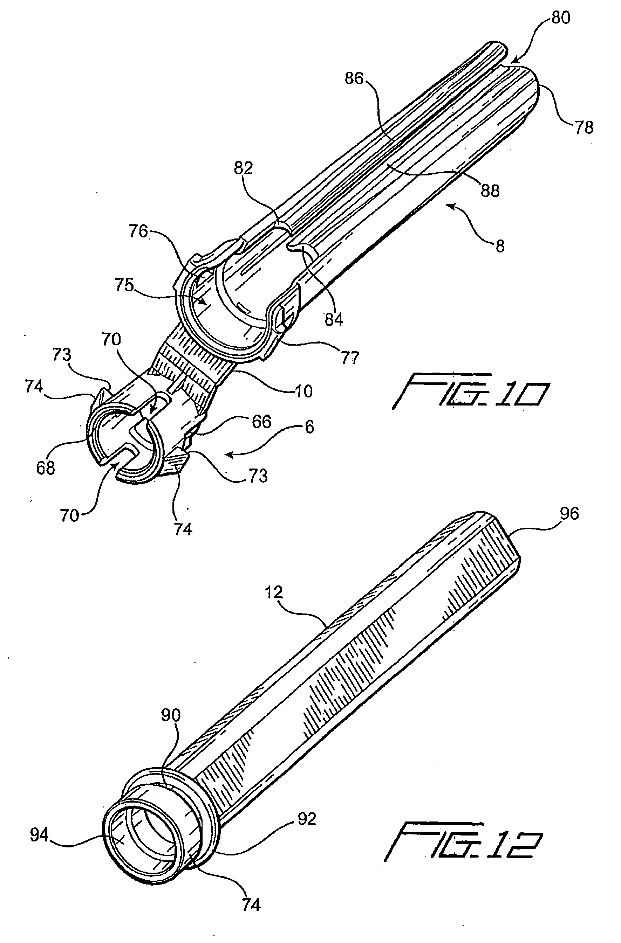

Opening 80, due to its formation by lips 82 and 84, is off-centered to one side of housing 8 to enhance the entry of needle 22 into housing 8. Each of lips 82 and 84 is angled, by a series of complex angles, as best shown in Figs. 10 and 11, toward the interior of housing 8. The respective angles of each of the lips are therefore varied along the length of the housing for guiding needle 22 into housing 8 via opening 80. The respective progressively angled surfaces of lips 82 and 84 are designated 86 and 88, respectively. Given that the entry of needle 22 into housing 8 is guided by lips 82 and 84, the angled entry of needle 22 into housing 8 is effected in a smooth manner to substantially eliminate the possibility that contaminated fluid that remains on needle 22 after its use may be flickered or splattered when needle 22 comes into contact with housing 8.

To ensure that needle protection housing 8 remains fixedly retained along the longitudinal axis 14, a lock mechanism is provided at the proximal end 75 of needle housing 8 and the outer surface of collar 6. This ensures that once needle housing 8 is pivoted to the position along longitudinal axis 14, it will remain in alignment thereat. This lock mechanism, as shown in Figs. 1-3 and 9-11, comprises two apertures 76 at the base of needle housing 8, and two corresponding one-way downward sloping catch members 74 at collar 6. Alternatively, as should readily be recognized, the apertures and catch members may be formed at collar 6 and the base of housing 8, respectively. Further, instead of apertures, non-through openings that nonetheless mate to the catch members are also envisioned. When needle protection housing 8 is pivoted to be in alignment along longitudinal axis 14, aperture 76 will snap fit over the one-way catch members 74, with the base surfaces 73 of the one-way catch members 74 acting against the top surfaces 77 at the base of apertures 76 to thereby fixedly retain needle housing 8 relative to collar 6.

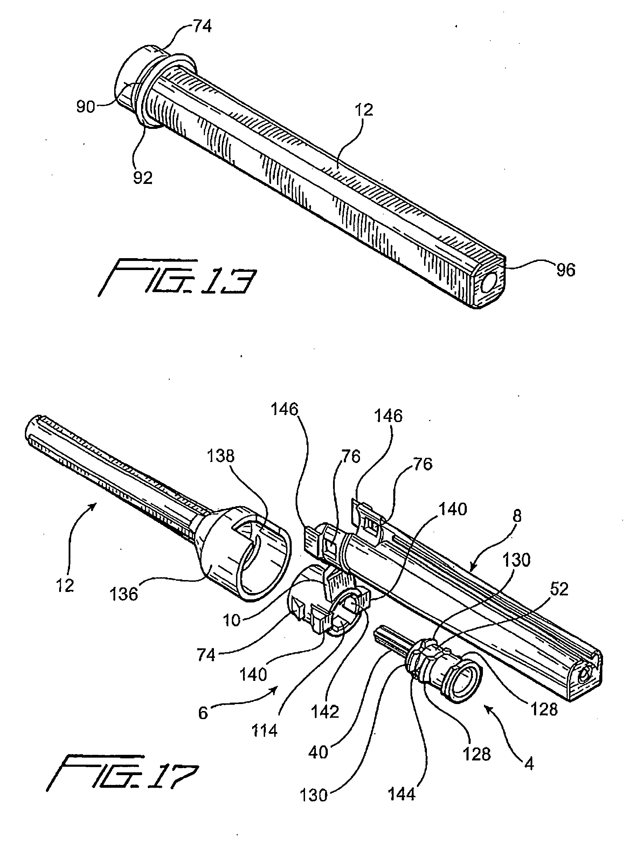

As shown in Figs. 12 and 13, needle sheath 12 has a first engage mechanism that engages to a second engage mechanism at collar 6. In particular, needle sheath 12 is a cylindrical cap that has formed at its proximal portion 74 a circumferential groove or slot 90. Groove 90 is configured to have a dimension, as defined by stop 92 and proximal portion 75, to accept rib 68 of collar 6. As shown, proximal portion 74 of needle sheath 12 has an opening 94 that allows needle sheath 12 to be placed or positioned over needle 22 and be press-fitted onto the distal end of collar 6. As the distal end of collar 6 has a rib 68 and opposed slots 70, when needle sheath 12 is fitted to collar 6, due to the elastic properties of the plastic material from which both collar 6 and needle sheath 12 are molded, the distal end of collar 6 would expand slightly so as to accept the proximal portion 75 of needle sheath 12, until rib 68 is snap fitted into groove 90, and the edge of the distal end of collar 6 rests against stop 92. Once snap fitted to collar 6, needle sheath 12 is removably engaged to collar 6. To remove needle sheath 12 from collar 6, a predetermined or greater force is applied to needle sheath 12 along longitudinal axis 14 for separating needle sheath 12 from collar 6. As best shown in Fig. 13, needle sheath 12 has a closed distal end 96.

In operation, with the assembled safety needle assembly as shown in Fig. 3, a user would remove needle sheath 12 by applying a predetermined force longitudinally relative to collar 6. Once exposed, needle 22 may be used. After use, needle protective housing 8 is pivoted to be in substantial alignment along longitudinal axis 14 so that the contaminated needle 22 enters into housing 8 and is trapped inside housing 8 by the trapdoor formed by lips 82 and 84. At the same time, housing 8 is fixedly retained to collar 6 by the mating of apertures 76 at the base of housing 8 to the one-way catch member 74 at the outer surface of collar 6. To remove needle hub 4 from the syringe, ring 28 of needle hub 4 is grasped, and in the case of a luer lock coupling, rotated counter-clockwise to remove needle hub 4 from the syringe, such as 16 shown in Fig. 2. Once removed from the syringe, the safety needle assembly could be properly disposes.

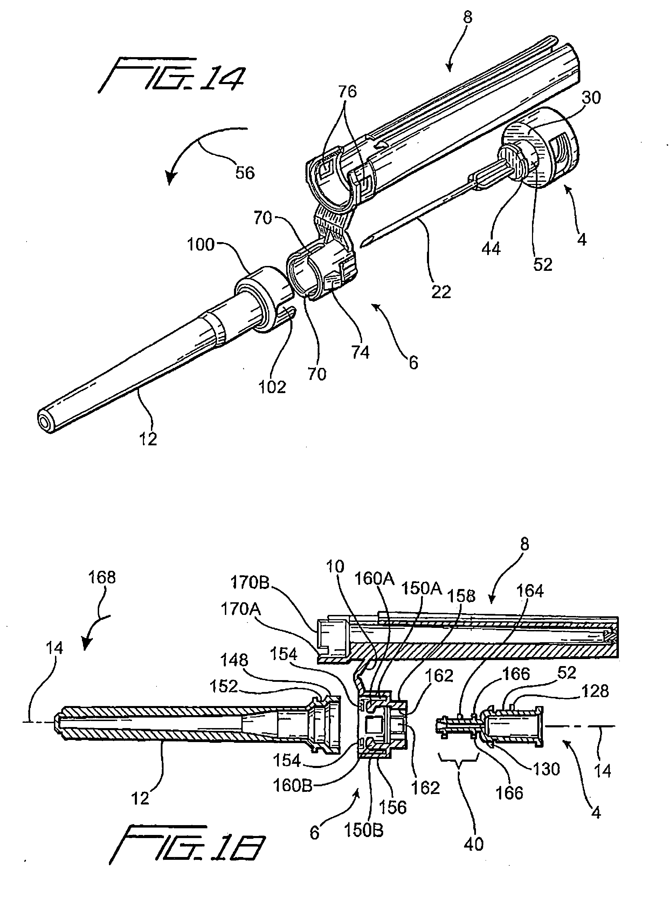

A first embodiment of the needle assembly of the instant invention is shown in Fig. 14. Elements that are the same or similar to those of the earlier embodiment are labeled the same. As shown, needle hub 4 of the Fig. 14 embodiment is the same as that shown in the earlier embodiment. Needle sheath 12, however, is a clear sheath or cap that does not come into contact with needle hub 4. In particular, needle sheath 12 is shown to have a base portion 100 that is dimensioned to have a diameter greater than the diameter of collar 6. Base portion 100 also is formed to include opposed slots or keys 102, of which only one is shown, that mate with catch members 74 (only one is shown) of collar 6. Thus, with collar 6 fitted about space or groove 52 defined between flanges 44 and end wall 30 of needle hub 4, collar 6 is rotatable about needle hub 4, when needle sheath 12 is rotated. Two opposed slits 70 are provided at the distal portion of collar 6 for providing flexibility thereat so that the needle sheath 12 can readily and tightly connect to collar 6. By being transparent, when collar 6 is seated onto groove 52 of needle hub 4 and needle sheath 12 slip-fitted over collar 6, a user can see needle 22, so as to readily determine the gauge of the needle. Once needle sheath 12 is removed from collar 6, catch members 74 would coact with the lock member apertures 76 at the base of housing 8 for fixedly retaining housing 8 to collar 6, when housing 8 is pivoted along direction 56 to cover needle 22. To connect and disconnect the needle assembly of the Fig.14 embodiment to a syringe, the same operation as noted for the previous embodiment, namely grasping the needle hub 4, is used to rotatably connect and disconnect the same from the luer end of a syringe.

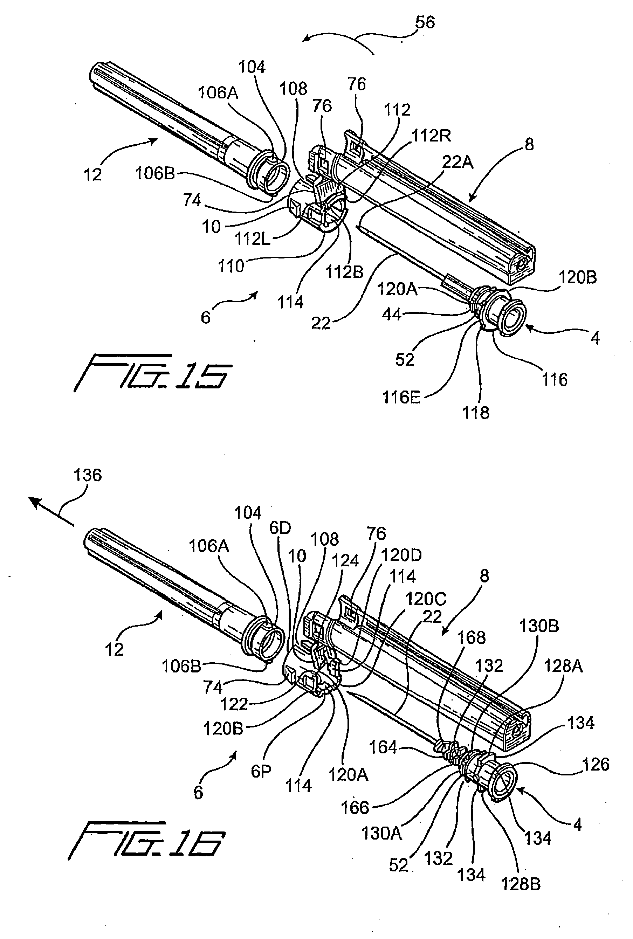

The Fig. 15 embodiment of the needle assembly of the instant invention has a needle sheath having a base portion 104 that is dimensioned to fit into the distal end of collar 6. There are provided at base portion 104 of needle sheath 12 two keys 106a and 106b which are matable with corresponding slots or channels 108 (only one is shown) provided at the distal portion of collar 6 Thus, with needle sheath 12 mated to collar 6, by means of keys 106a and 106b being mated to keyways 108, collar 6 is rotatable in unison with needle sheath 12.

For the collar 6 of the Fig. 15 embodiment, there is provided at its proximal end 110 a flange 112 proximate to living hinge 10. As with the previous embodiments, there are provided within the proximal portion of collar 6 a plurality of pads 114 which are movably mounted to space or groove 52 of needle hub 4, so as to allow collar 6 to be rotatable about needle hub 4. For the Fig. 15 embodiment, a ring 116 is provided as the back end of groove 52, whereas a plurality of flanges 44 define the front end of groove 52. Ring 116 is designed to have two end stops 118 (only one being shown) that are at 180° from each other. There are also provided on the top edge of ring 116 two protuberances 120a and 120b.

With collar 6 mounted to needle hub 4, and in particular pads 114 movably resting on groove 52, the bottom surface 112b of flange 112 comes into contact with edge 116e of ring 116. With the end stops 118 at the 3 and 9 o'clock positions, collar 6 is rotatable between those end stops 118, so as to ensure that the beveled end 22a of needle 22 can readily be seen by the user when the user inserts needle 22 into the vein of a patient. To align housing 8 at the 12 o'clock position, collar 6 is rotated until flange 112 is positioned between protuberances 120a and 120b. Flange 112 is able to be snap-fitted between protuberances 120a and 120b due to the respective elastic characteristics of flange 112 and the protuberances, as both collar 6 and needle hub 4 are made from a conventional medical plastics material that does have a given elasticity.

With ring 116, and the interaction of flange 112 with the two end stops 118, once collar 6 is mounted to needle hub 4 and needle sheath 12 mated to collar 6, by turning needle sheath 12, assuming a clockwise movement, the left end 112l of flange 112 eventually comes into contract with end stop 118 to thereby cause needle hub 4 to turn when collar 6 is rotated. Similarly, when collar 6 is turned counterclockwise, (looking in the direction of needle hub 4), end 112r of flange 112 eventually comes into contact with the not shown end stop 118 so as to cause needle hub 4 to rotate counterclockwise.

To use the Fig. 15 needle assembly, before exposing needle 22, a user would rotate needle sheath 12 in a clockwise direction, so that collar 6, with flange 112, causes needle hub 4 to rotate in a clockwise direction to connect to a syringe. After needle sheath 12 is removed to expose needle 22, to cover needle 22, housing 8 is pivoted in the direction of arrow 56 to cover needle 22. Once needle 22 is stored within housing 8 and catch members 74 coact with lock members 76, a user could simply turn housing 8 in a counterclockwise direction so that flange 112 would abut against the not shown end stop of needle hub 4 to thereby cause needle 4 to rotated in a counterclockwise direction, thereby removing needle hub 4 and therefore the needle assembly from the syringe.

The needle assembly of the Fig. 16 embodiment has a needle sheath 12 that is the same as that of the Fig. 15 embodiment in that it has a base portion 104 that has two opposed keys 106a and 106b at the distal end of collar 6. The collar 6 of the Fig. 16 embodiment has a proximal portion that has at the proximal end a plurality of fingers, in this instance four, designated 120a-120d. These fingers or extensions are interspersed with internal pads 114 at its proximal portion of collar 6. There is formed at each of the internal pads 114 a head or extension 122. Further with respect to collar 6, note that keyway 108 as shown extends from the distal end 6d of collar 6 to its proximal end 6p and then ends at a portion 124 of living hinge 10, which extends from the proximal portion of collar 6.

The needle hub 4 of the Fig. 16 embodiment has a luer end 126 and two sets of flanges orthogonally extending from its main body for defining the space or groove 52 onto which internal pads 114 of collar 6 are mounted. The pair of flanges that are shown in the form of wings are designated 128a and 128b and may be referred to as the aft flanges. The other set of flanges, which may be referred to as the fore flanges, are made up of a number of adjacent flanges 130, of which only 130a and 130b are fully shown. The space defined between the adjacent flanges, for example that between flanges 130a and 130b, are designated 132.

With collar 6 rotatably mounted about needle hub 4, with internal pads 14 movably mounted on groove 52, to connect luer end 126 of needle hub 4 to a counterpart end of a syringe, a user would hold needle sheath 12, which is mated to the distal portion of collar 6, and rotate clockwise while pushing in at the same time. Due to the interlocking between keys 106 of needle sheath 12 with keyways 108 of collar 6, collar 6 is rotated in unison with needle sheath 12. By pushing needle sheath 12 toward needle hub 4, collar 6 is likewise moved forward toward needle hub 4, with corresponding pairs of fingers 120 extending between the edges of flanges 128a and 128b, designated as 134. As a consequence, when needle sheath 12 is rotated clockwise, one of the fingers 120 would come into contact with the edge of flange 128b while another one of the fingers 120 would come into contact with an opposite edge of flange 128a to thereby cause needle hub 4 to be rotated in unison with collar 6, as needle sheath 12 is being turned. The needle assembly is thus connected to a syringe.

To disconnect the needle assembly from the syringe, after the needle sheath 12 has been removed from collar 6 to expose needle 22, and with housing 8 pivoted to cover contaminated needle 22 and fixedly retained to collar 6 by the locking mechanism comprising catch members 74 at collar 6 and apertures 76 at housing 8, a user would pull housing 8 in the direction indicated by directional arrow 136 to thereby move collar 6 in a direction away from needle hub 4. When thus moved, the heads 122 extending from the internal pads 114 would eventually be fitted to the spaces 132 defined by adjacent flanges 130. Once heads 122 are caught in their respective spaces 132, by turning housing 8 in a counterclockwise direction, needle hub 4 likewise is moved in the same counterclockwise direction to thereby disconnect the needle assembly from the luer of the syringe.

Fig. 17 illustrates yet another embodiment of the needle assembly of the instant invention. For the Fig. 17 embodiment, needle sheath 12 is shown to include a proximal portion 136 that has a dimension that allows it to fit over collar 6. For illustration purpose, the needle that is attached to the distal end 4d of the needle hub is not shown. For the Fig. 17 embodiment, needle hub 4 has a main body and, as was the case with the Fig. 16 embodiment, two sets of flanges that are orthogonally extending from the main body portion. The first set of flanges may be considered as the aft flanges 128 while the second set of flanges may be considered as the fore flanges 130.

The base portion 136 of needle sheath 12 is enlarged, with a dimension that allows it to fit over collar 6. A channel 138 is provided at portion 136 and is dimensioned to allow it to mate with the portion of living hinge 10 that extends from collar 6. Thus, for the Fig. 17 embodiment, collar 6 is substantially fitted within base portion 136 of needle sheath 12 before use.

The Fig. 17 collar 6, in addition to having the catch members 74, has connected thereto housing 8 by means of living hinge 10. Collar 6 further includes, a plurality of spring arms 140, in this instance two, each having an internal pad 142 that is similar to the integral internal pad 114 at the inner wall of the proximal portion of collar 6 interspersed between the spring arms 140. As shown, each of the spring arms 140 is configured to have a bevel front end that allows portion 136 of needle sheath 12 to easily fit over. When the inner wall of portion 136 comes into contact with the outer surface of spring arms 140, spring arms 140 are pushed inwards toward the center axis of collar 6. As spring arms 140 are pushed inwards, their respective inner pads 142 eventually come to rest when pressed against corresponding flat sections 144 (only one of which is shown) formed at groove 52 of needle hub 4. Spring arms 140, by way of their respective internal pads 142, therefore act to hold needle hub 4, when needle sheath 12 is slip fitted over collar 6. As a consequence, the needle assembly of Fig. 17 may be connected to a syringe when a user rotates needle sheath 12 in a direction, for example clockwise, that threadingly mates needle hub 4 to the luer end of a syringe.

To use, needle sheath 12 is removed with a predetermined force from collar 6. After use, to cover the contaminated needle, housing 8 is pivoted to cover the now contaminated needle, with catch members 74 of collar 6 coacting with apertures 76 of housing 8 to fixedly retain housing 8 to collar 6.

For the Fig. 17 embodiment, at the base of housing 8 adjacent to apertures 76 there are two legs or extensions that extend from the open end of housing 8. When housing 8 is pivoted to the position whereby it is in substantial alignment along the longitudinal axis of the needle for covering the needle, legs 146 would come into contact with spring arms 140 to bias spring arms 140 inwardly to again press against the corresponding flat sections 144 at needle hub 4. As a consequence, collar 6 once more fixedly holds needle hub 4 when housing 8 has been pivoted to cover the needle. At that point, to remove the needle assembly from the syringe, a user only needs to turn housing 8 in the appropriate direction, for example the counterclockwise direction, as needle hub 4 is rotated in unison with collar 6 when housing 8 is rotated.

Fig. 18 is a cross-sectional view of yet another embodiment of the needle assembly of the instant invention. For the Fig. 18 embodiment, needle sheath 12 has a base portion that has an opening that is dimensioned to fit into collar 6. In particular, needle sheath 12 has a contact portion 148 that fits within space 150 when needle sheath 12 is snap fitted to collar 6. Needle sheath 12 is held to collar 6 by the interaction between groove 152 and fingers 154 provided internally at the distal end of collar 6.

As shown, collar 6 has a distal portion 156 and a proximal portion 158, which has a smaller diameter than distal portion 156. There are a plurality of spring fingers 160 that extend from proximal portion 158 into the interior of distal portion 156 of collar 6. When needle sheath 12 is snap-fitted into collar 6, portion 148 thereof would bias against spring fingers 160 to force them to move toward the center of collar 6. A number of inner pads 162 provided at proximal portion 158 of collar 6 are mounted about groove 52 of needle hub 4, so that, if spring fingers 160 are left at their natural position, collar 6 is rotatable about needle hub 4.

Needle hub 4 of the Fig. 18 embodiment is the same as the needle hub shown in the Fig. 16 embodiment. For the Fig. 18. embodiment, there are a number of sets of concentric arms 164, 166 provided at distal portion 4d of needle hub 4. As best shown in Fig. 16, adjacent pairs of these arms define a V space 168 that the compressed spring fingers 160 would be engaged with, when the spring fingers 160 are pushed inward by the mating of needle sheath 12 to collar 6 in the manner as described earlier. Thus, with collar 6 rotatably mounted to needle hub 4 and needle sheath 12 snap-fitted to collar 6, the spring fingers 160 of collar 6 would fixedly hold needle hub 4, so that needle hub 4 and collar 6 would rotate in unison, when a user turns needle sheath 12, which has its portion 148 tightly and frictionally fitted within space 150 of collar 6.

As in all of the embodiments, once collar 6 is rotatably mounted to groove 52 of needle hub 4, to remove needle sheath 12, the user applies a predetermined force sufficient to separate portion 148 of needle sheath 12 from space 150 and groove 152 from fingers 154. Once needle sheath 12 is removed from collar 6, collar 6 is rotatable about needle hub 4 so that housing 8 may be positioned to a location that does not conflict with the viewing of the bevel tip of the needle. After use, to cover the contaminated needle, which for the Fig. 18 embodiment is not shown for ease of explanation, housing 8 is pivoted in the direction as shown by directional arrow 168 so that it becomes substantially in alignment along the longitudinal axis 14 of the needle assembly and be fixedly retained by the coaction of the catch members 74 of collar 6 (not shown) and apertures 76 (not shown) at housing 8.

For the Fig. 18 embodiment, hosing 8 has extending at its open end a number of legs or extensions 170 that, when housing 8 is pivoted to the position in alignment with longitudinal axis 14, would enter the interior of the distal portion 156 of collar 6, so that the extensions 170, for example extension 170a would fit within space 150 of collar 6 to thereby bias spring fingers 160 inwardly. Spring fingers 160 in turn would fit within one of the spaces 168 defined by adjacent arms 164 or 166, so that needle hub 4 is fixedly held to collar 6. Thus held, when a user rotates housing 8, for example in the counterclockwise direction, collar 6 is rotated in the same direction to thereby disconnect needle hub 4 from a syringe.

## Claims

1. A safety apparatus, comprising:
a needle hub (4)having a proximal portion (18) and a distal portion (20), a needle (22) extending from a distal end of said needle hub (4);
a collar (6) rotatably mounted about the distal portion (20) of said needle hub (4);
a housing (8) pivotally connected to said collar (6); and
a needle sheath (12) collar (6)for covering said needle (22) extending from the distal end of said needle hub (4) **characterised in that** the needle sheath (12) is attached to the collar (6) such that the needle sheath does not come into contact with the needle hub (4).

2. A safety apparatus of claim 1, wherein after said needle sheath is removed from said collar (6), said housing (8) is pivotable to a position substantially in alignment along a longitudinal axis of said needle hub (4) for covering said needle (22).

3. A safety apparatus of claim 1, wherein said needle sheath (12) has a first engage mechanism (90) proximate to its open end (94) and wherein said collar (6) has a second engage mechanism (68) formed at its distal portion; and
wherein when said needle sheath (12) is positioned over said needle (22) and mates with said collar (6), said first engage mechanism (90) engages (90) with said second engage mechanism (68) to attach said needle sheath (12) to said collar (6).

4. A safety apparatus of claim 1, wherein said needle sheath (12) has a groove (90) formed circumferentially therearound proximate to its open end (94) and wherein said collar (6) has a rib (68) circumferentially formed at the inner wall of its distal end; and
wherein said needle sheath (12) is attached to said collar (6) when said rib of said collar (6) mates with said groove after said needle sheath (12) is positioned over said needle (22) and engages said collar (6).

5. A safety apparatus of claim 1, wherein said needle hub (4)comprises a luer end (40) at its proximal portion (18), a ring (28) surrounding and spaced from said luer end, said ring being integral of said needle hub (4) via a distal end wall (30) extending transversely therefrom; and
wherein a user can readily grasp said ring to couple said safety device to a medical device by threadingly mating said luer end of said needle hub (4) to a counterpart luer end at said medical device.

6. A safety apparatus of claim 5, wherein said ring comprises at least one window (42) to enable the user to view said luer end of said needle hub (4) and said needle hub (4).

7. A safety apparatus of claim 1, wherein said needle hub (4) comprises at least one flange (44) extending from its distal portion, said flange (44) being located a predetermined distance from a wall (30) projecting orthogonally from said needle hub, a space (52) being formed between said flange (44) and said wall circumferentially about said needle hub (4); and
wherein said collar (6) comprises at least one protrusion (62) at the inner wall of its proximal portion (18), said protrusion (62) being dimensioned to fit to said space defined between said flange (44) and said wall when said collar (6) is mated to said needle hub (4), said collar (6) rotatable about said needle hub (4) after matingly fitted to said space.

8. A safety apparatus of claim 1, wherein said housing (8) comprises a longitudinal opening (80) formed by first and second lips (82, 84) each extending along substantially the length of said housing (8), said first lip (82) overlapping a portion of said second lip (84) with said opening (80) being off centered from said longitudinal axis, each of said lips (82, 84) being angled toward the interior of said housing (8) with the respective angles of said lips (82, 84) being varied along the length of said housing (8) to effect a guide for said needle (22) to smoothly enter into said housing (8) at an angle through said opening (80) when said housing (8) is pivoted to cover said needle (22), said needle (22) not being removable from said housing once said needle (22) fully enters into said housing (8).

9. A safety apparatus of claim 1, wherein said collar (6) has formed at its outer surface one lock mechanism (74) and wherein said housing (8) has formed at its proximal end an other lock mechanism (76), said one and other lock mechanisms (76) coacting to fixedly retain said housing to said collar (6) once said housing (8) is pivoted to a position in substantial alignment with said needle hub (4) to cover said needle (22).

10. A safety apparatus of claim 9, wherein said one lock mechanism comprises at least one one way catch member (74) extending from the outer surface of said collar (6) or said housing (8), and said other lock mechanism (76) comprises at least one corresponding aperture (76) at said housing or said collar (6), said one way catch member (74) matingly coupled to said aperture (76) for fixedly retaining said housing (8) to said collar (6) when said housing (8) is pivoted to cover said needle (22).

11. In combination, a needle hub (4) having a proximal portion (18) and a distal portion (20), said proximal portion (18) having a luer connector (40) and a ring (28) surrounding but in spaced relationship with said luer connector (40), said distal portion (20)of said needle hub (4) having a distal end from which a needle (22) extends, a collar (6) having a housing (8) pivotally connected thereto fitted to and rotatable about said distal portion (20) of said needle hub (4).

12. A combination of claim 11, further comprising a needle sheath (12) connected to said collar (6), said needle sheath (12) removable from said collar (6) to expose said needle (22) for use.

13. A combination of claim 12, wherein said needle sheath (12) comprises a first engage mechanism (90) proximate to its open end (94) and wherein said collar (6) comprises a second engage mechanism (68) at its distal portion, said first engage mechanism (90) engages said second engage mechanism (68) for attaching said needle sheath (12) to said collar (6) when said needle sheath (12) is positioned over said needle and mates with said collar (6).

14. A combination of claim 12, wherein said needle sheath (12) comprises a circumferential groove (90) proximate to its open end (94) and said collar (6) comprises a circumferential rib (68) at its distal portion, said rib mating to said groove when said needle sheath (12) is positioned over said needle (12) and fitted to said collar (6).

15. A combination of claim 11, wherein said ring (28) comprises at least one window (42) on its sidewall (32) to enable viewing of said luer connector and said proximal portion (18) of said needle hub (4).

16. A combination of claim 11, wherein said ring is adaptable to be used by a user to grasp said needle hub (4)for connecting said luer connector (40) to a corresponding luer connector of a medical device.

17. A combination of claim 11, wherein said needle hub (4) comprises a plurality of flanges (44) extending from its distal portion (20), said flanges (44) being located a predetermined distance from a wall (30) projecting orthogonally from said needle hub (4), a space (52) being defined between said flanges (44) and said wall circumferentially about said needle hub (4), and wherein said collar (6) comprises a plurality of protrusions (62) at the inner wall of its proximal portion (18), said protrusions (62) being dimensioned to fit to said space when said collar (6) is mated to said needle hub (4), said collar (6) rotatable about said needle hub (4) after matingly fitted to said space.

18. A combination of claim 11, wherein said housing (8) comprises a longitudinal opening (80) formed by first and second lips (82, 84) each extending along substantially the length of said housing, said first lip (82) overlapping a portion of said second lip (84) with said opening being off centered from said longitudinal axis, each of said lips (82, 84) being angled toward the interior of said housing (8) with the respective angles of said lips (82, 84) being varied along the length of said housing (8) to effect a guide for said needle (22) to smoothly enter into said housing (8) at an angle through said opening (80) when said housing (8) is pivoted to cover said needle (22), said needle (22) not removable from said housing (8) once said needle (22) fully enters into said housing (8).

19. A combination of claim 11, wherein said collar (6) has formed at its outer surface a first lock mechanism (74) and wherein said housing (8) has formed at its proximal end a second lock mechanism (76), said first and second lock mechanisms (74, 76) coacting to fixedly retain said housing to said collar (6) once said housing (8) is pivoted to a position in substantial alignment with said needle hub (4) to cover said needle (22).

20. A method of making a needle assembly, comprising the steps of:
a) providing a needle hub (4) having a proximal portion (18) and a distal portion (20);
b) fixedly attaching a needle (22) to a distal end of said needle hub (4);
c) pivotally connecting housing (8) to a collar (6);
d) rotatably mounting said collar (6) about the distal portion (20) of said needle hub (4); and **characterised by**
e) attaching a needle (22) sheath (12) to said collar (6) such that the needle sheath does not come into contact with the needle hub (4) for covering said needle (22) extending from the distal end of said needle hub.

21. A method of claim 20, further comprising the steps of:
removing said needle sheath (12) from said collar (6) before using said needle (22); and
pivoting said housing (8) to a position substantially in alignment along a longitudinal axis of said needle hub (4) for covering said needle (22).

22. A method of claim 20, further comprising the steps of:
forming a first engage mechanism (90) on said needle sheath (12) proximate to its open end (94);
forming a second engage mechanism (68) on a distal portion (20) of said collar (6); and
wherein said step e comprises the steps of:
positioning said needle sheath (12) over said needle (22); and
mating said needle sheath (12) with said collar (6) until said first engage mechanism (90) engages said second engage mechanism (68).

23. A method of claim 20, further comprising the steps of:
forming a circumferential groove (90) proximate to an open end (94) of said needle sheath (12);
forming a rib (68) circumferentially at the inner wall of a distal portion (20) of said collar (6); and
wherein said step e comprises the steps of:
positioning said needle sheath (12) over said needle (22); and
engaging said needle sheath (12) to said collar (6) until said rib of said collar (6) mates with said groove of said needle sheath (12).

24. A method of claim 20, wherein said step a comprises the steps of:
forming a luer end (40) at the proximal portion (18) of said needle hub (4); and
forming a ring (28) in spaced relation to surround said luer end (40), said ring (28) being integral of said needle hub (4) via a distal end wall (30);
wherein a user can readily grasp said ring to couple said safety device to a medical device by threadingly mating said luer (40) end of said needle hub (4) to a counterpart luer end at said medical device.

25. A method of claim 24, wherein said forming a ring (28) step further comprises the step of:
forming at least one window (42) on said ring (28) to enable the user to view said luer end (40) of said needle hub (4) and said needle hub (4).

26. A method of claim 20, wherein said step a comprises the steps of:
providing at least one flange (44) extending from said distal portion (20) of said needle hub (4); and
locating said flange (44) a predetermined distance from a wall (30) projecting orthogonally from said needle hub (4) to define a space (52) between said flange (44) and said wall circumferentially about said needle hub (4);
wherein the method further comprising the steps of:
forming at least one protrusion (62) at the inner wall of said collar (6);
dimensioning said protrusion (62) to fit to said space defined between said flange (44) and said wall; and
mating said collar (6) to said needle hub (4), said collar (6) rotatable about said needle hub (4) after being mated to said space.

27. A method of claim 20, further comprising the step of:
providing a longitudinal opening (80) along said housing (8) by forming first and second lips (82, 84) each extending along substantially the length of said housing (8), said first lip (82) overlapping a portion of said second lip (24) with said opening (80) being off centered from said longitudinal axis, each of said lips (82, 84) being angled toward the interior of said housing (8) with the respective angles of said lips (82, 84) being varied along the length of said housing (8) to effect a guide for said needle (22) to smoothly enter into said housing (8) at an angle through said opening (80) when said housing (8) is pivoted to cover said needle (22), said needle (22) not removable from said housing (8) once said needle (22) fully enters into said housing (8).

28. A method of claim 20, further comprising the steps of:
forming a first lock mechanism (74) at the outer surface of said collar (6); and
forming a second lock mechanism (76) at a proximal end of said housing (8);
wherein said first and second lock mechanisms (74, 76) coact to fixedly retain said housing (8) to said collar (6) once said housing (8) is pivoted to a position in substantial alignment with said needle hub (4) to cover said needle (22).

## Patentansprüche

1. Sicherheitsvorrichtung, die Folgendes umfasst:
eine Nadelnabe (4) mit einem proximalen Abschnitt (18) und einem distalen Abschnitt (20), eine Nadel (22), die sich von einem distalen Ende der genannten Nadelnabe (4) erstreckt;
einen Bund (6), der drehbar um den distalen Abschnitt (20) der genannten Nadelnabe (4) montiert ist;
ein Gehäuse (8), das schwenkbar mit dem genannten Bund (6) verbunden ist; und
einen Nadelschutzkappen-(12)-Bund (6) zum Abdecken der genannten Nadel (22), die sich vom distalen Ende der genannten Nadelnabe (4) erstreckt, **dadurch gekennzeichnet, dass** die Nadelschutzkappe (12) so an dem Bund (6) angebracht ist, dass sie nicht mit der Nadelnabe (4) in Kontakt kommt.

2. Sicherheitsvorrichtung nach Anspruch 1, wobei das genannte Gehäuse (8) nach dem Abnehmen der genannten Nadelschutzkappe von dem genannten Bund (6) in eine Position gedreht werden kann, in der es im Wesentlichen entlang einer Längsachse der genannten Nadelnabe (4) ausgerichtet ist, um die genannte Nadel (22) zu bedecken.

3. Sicherheitsvorrichtung nach Anspruch 1, wobei die genannte Nadelschutzkappe (12) einen ersten Eingriffsmechanismus (90) in der Nähe ihres oberen Endes (94) hat und wobei der genannte Bund (6) einen zweiten Eingriffsmechanismus (68) hat, der an seinem distalen Abschnitt ausgebildet ist; und
wobei der genannte erste Eingriffsmechanismus (90), wenn die genannte Nadelschutzkappe (12) über der genannten Nadel (22) positioniert und mit dem genannten Bund (6) zusammengesteckt ist, in den genannten zweiten Eingriffsmechanismus (68) eingreift, um die genannte Nadelschutzkappe (12) an dem genannten Bund (6) anzubringen.

4. Sicherheitsvorrichtung nach Anspruch 1, wobei die genannte Nadelschutzkappe (12) eine Nut (90) aufweist, die in der Nähe ihres offenen Endes (94) umfangsmäßig darum herum ausgebildet ist, und wobei der genannte Bund (6) eine Rippe (68) hat, die umfangsmäßig an der Innenwand seines distalen Endes ausgebildet ist; und
wobei die genannte Nadelschutzkappe (12) an dem genannten Bund (6) angebracht ist, wenn die genannte Rippe des genannten Bunds (6) nach dem Setzen der genannten Nadelschutzkappe (12) auf die genannte Nadel (22) mit der genannten Nut zusammengesteckt ist und in den genannten Bund (6) eingreift.

5. Sicherheitsvorrichtung nach Anspruch 1, wobei die genannte Nadelnabe (4) ein Luer-Ende (40) an ihrem proximalen Abschnitt (18) aufweist, wobei das genannte Luer-Ende von einem Ring (28) in einem Abstand davon umgeben ist, wobei der genannte Ring mit der genannten Nadelnabe (4) über eine sich transversal davon erstreckende distale Endwand (30) einstückig ausgebildet ist; und
wobei ein Benutzer den genannten Ring leicht ergreifen kann, um die genannte Sicherheitsvorrichtung mit einer medizinischen Vorrichtung durch Schrauben des genannten Luer-Endes der genannten Nadelnabe (4) auf ein Luer-Gegenende an der genannten medizinischen Vorrichtung zu koppeln.

6. Sicherheitsvorrichtung nach Anspruch 5, wobei der genannte Ring wenigstens ein Fenster (42) aufweist, so dass der Benutzer das genannte Luer-Ende der genannten Nadelnabe (4) und die genannte Nadelnabe (4) sehen kann.

7. Sicherheitsvorrichtung nach Anspruch 1, wobei die genannte Nadelnabe (4) wenigstens einen Flansch (44) umfasst, der sich von seinem distalen Abschnitt erstreckt, wobei sich der genannte Flansch (44) in einem vorbestimmten Abstand von einer Wand (30) befindet, die orthogonal von der genannten Nadelnabe vorsteht, wobei ein Raum (52) zwischen dem genannten Flansch (44) und der genannten Wand umfangsmäßig um die genannte Nadelnabe (4) ausgebildet ist; und
wobei der genannte Bund (6) wenigstens einen Vorsprung (62) an der Innenwand seines proximalen Abschnitts (18) umfasst, wobei der genannte Vorsprung (62) so dimensioniert ist, dass er zu dem genannten Raum passt, der zwischen dem genannten Flansch (44) und der genannten Wand definiert ist, wenn der genannte Bund (6) mit der genannten Nadelnabe (4) zusammengesteckt ist, wobei der genannte Bund (6) nach dem Einstecken in den genannten Raum um die genannte Nadelnabe (4) drehbar ist.

8. Sicherheitsvorrichtung nach Anspruch 1, wobei das genannte Gehäuse (8) eine Längsöffnung (80) umfasst, die von einer ersten und einer zweiten Lippe (82, 84) gebildet wird, die jeweils entlang im Wesentlichen der Länge des genannten Gehäuses (8) verlaufen, wobei die genannte erste Lippe (82) einen Abschnitt der genannten zweiten Lippe (84) überlappt, wobei die genannte Öffnung (80) exzentrisch von der genannten Längsachse ist, wobei jede der genannten Lippen (82, 84) in Richtung auf das Innere des Gehäuses (8) abgewinkelt ist, wobei sich die jeweiligen Winkel der genannten Lippen (82, 84) entlang der Länge des genannten Gehäuses (8) ändern, um eine Führung für die genannte Nadel (22) zu erzielen, so dass diese leichtgängig in das genannte Gehäuse (8) in einem Winkel durch die genannte Öffnung (80) eintreten kann, wenn das genannte Gehäuse (8) gedreht wird, um die genannte Nadel (22) zu bedecken, wobei die genannte Nadel (22) nach ihrem völligen Eintreten in das genannte Gehäuse (8) nicht von dem genannten Gehäuse abgenommen werden kann.

9. Sicherheitsvorrichtung nach Anspruch 1, wobei auf der Außenfläche des genannten Bunds (6) ein Arretiermechanismus (74) ausgebildet ist und wobei am proximalen Ende des genannten Gehäuses (8) ein anderer Arretiermechanismus (76) ausgebildet ist, wobei der genannte eine und der andere Arretiermechanismus (76) zusammenwirken, um das genannte Gehäuse fest an dem genannten Bund (6) zu halten, wenn das genannte Gehäuse (8) in eine Position gedreht wird, in der es im Wesentlichen auf die genannte Nadelnabe (4) ausgerichtet ist, um die genannte Nadel (22) zu bedecken.

10. Sicherheitsvorrichtung nach Anspruch 9, wobei der genannte eine Arretiermechanismus wenigstens ein Einweg-Rastelement (74) umfasst, das sich von der Außenfläche des genannten Bunds (6) oder dem genannten Gehäuse (8) erstreckt, und der genannte andere Arretiermechanismus (76) wenigstens ein entsprechendes Loch (76) an dem genannten Gehäuse oder dem genannten Bund (6) umfasst, wobei das genannte Einweg-Rastelement (74) mit dem genannten Loch (76) zusammengesteckt wird, um das genannte Gehäuse (8) fest an dem genannten Bund (6) zu halten, wenn das genannte Gehäuse (8) gedreht wird, um die genannte Nadel (22) zu bedecken.

11. Kombination aus einer Nadelnabe (4) mit einem proximalen Abschnitt (18) und einem distalen Abschnitt (20), wobei der genannte proximale Abschnitt (18) einen Luer-Verbinder (40) und einen Ring (28) hat, der den genannten Luer-Verbinder (40) umgibt, aber davon beabstandet ist, wobei der genannte distale Abschnitt (20) der genannten Nadelnabe (4) ein distales Ende hat, von dem sich eine Nadel (22) erstreckt, wobei ein Bund (6) ein Gehäuse (8) umfasst, das schwenkend damit verbunden, daran montiert und um den genannten distalen Abschnitt (20) der genannten Nadelnabe (4) drehbar ist.

12. Kombination nach Anspruch 11, die ferner eine Nadelschutzkappe (12) umfasst, die mit dem genannten Bund (6) verbunden ist, wobei die genannte Nadelschutzkappe (12) von dem genannten Bund (6) entfernt werden kann, um die genannte Nadel (22) für den Gebrauch zu exponieren.

13. Kombination nach Anspruch 12, wobei die genannte Nadelschutzkappe (12) einen ersten Eingriffsmechanismus (90) in der Nähe ihres offenen Endes (94) umfasst und wobei der genannte Bund (6) einen zweiten Eingriffsmechanismus (68) an seinem distalen Abschnitt umfasst, wobei der genannte erste Eingriffsmechanismus (90) in den genannten zweiten Eingriffsmechanismus (68) eingreift, um die genannte Nadelschutzkappe (12) an dem genannten Bund (6) anzubringen, wenn die genannte Nadelschutzkappe (12) über der genannten Nadel positioniert und mit dem genannten Bund (6) zusammengesteckt wird.

14. Kombination nach Anspruch 12, wobei die genannte Nadelschutzkappe (12) eine Umfangsnut (90) in der Nähe ihres offenen Endes (94) umfasst und der genannte Bund (6) eine Umfangsrippe (68) an seinem distalen Abschnitt umfasst, wobei die genannte Rippe in der genannten Nut steckt, wenn die genannte Nadelschutzkappe (12) über der genannten Nadel (12) positioniert und auf den genannten Bund (6) gesetzt wird.

15. Kombination nach Anspruch 11, wobei der genannte Ring (28) wenigstens ein Fenster (42) an seiner Seitenwand (32) umfasst, damit der genannte Luer-Verbinder und der genannte proximale Abschnitt (18) der genannten Nadelnabe (4) sichtbar sind.

16. Kombination nach Anspruch 11, wobei der genannte Ring so angepasst werden kann, dass er von einem Benutzer zum Ergreifen der genannten Nadelnabe (4) benutzt werden kann, um den genannten Luer-Verbinder (40) mit einem entsprechenden Luer-Verbinder einer medizinischen Vorrichtung zu verbinden.

17. Kombination nach Anspruch 11, wobei die genannte Nadelnabe (4) mehrere Flansche (44) umfasst, die sich von ihrem distalen Abschnitt (20) erstrecken, wobei sich die genannten Flansche (44) in einem vorbestimmten Abstand von einer Wand (30) befinden, die orthogonal von der genannten Nadelnabe (4) vorsteht, wobei ein Raum (52) zwischen den genannten Flanschen (44) und der genannten Wand umfangsmäßig um die genannte Nadelnabe (4) herum definiert ist und wobei der genannte Bund (6) mehrere Vorsprünge (62) an der Innenwand seines proximalen Abschnitts (18) umfasst, wobei die genannten Vorsprünge (62) so dimensioniert sind, dass sie zu dem genannten Raum passen, wenn der genannte Bund (6) mit der genannten Nadelnabe (4) zusammengesteckt wird, wobei der genannte Bund (6) nach dem Einstecken in dem genannten Raum um die genannte Nadelnabe (4) drehbar ist.

18. Kombination nach Anspruch 11, wobei das genannte Gehäuse (8) eine Längsöffnung (80) umfasst, die von einer ersten und zweiten Lippe (82, 84) gebildet wird, die jeweils über im Wesentlichen die Länge des genannten Gehäuses verlaufen, wobei die genannte erste Lippe (82) einen Abschnitt der genannten zweiten Lippe (84) überlappt, wobei die genannte Öffnung von der genannten Längsachse exzentrisch ist, wobei jede der genannten Lippen (82, 84) in Richtung auf das Innere des genannten Gehäuses (8) abgewinkelt ist, wobei sich die jeweiligen Winkel der genannten Lippen (82, 84) entlang der Länge des genannten Gehäuses (8) ändern, um eine Führung für die genannte Nadel (22) zu erzielen, so dass diese in das genannte Gehäuse (8) in einem Winkel leichtgängig durch die genannte Öffnung (80) eintreten kann, wenn das genannte Gehäuse (8) gedreht wird, um die genannte Nadel (22) zu bedecken, wobei die genannte Nadel (22) nach ihrem völligen Eintreten in das genannte Gehäuse (8) nicht von dem genannten Gehäuse (8) abgenommen werden kann.

19. Kombination nach Anspruch 11, wobei auf der Außenfläche des genannten Bunds (6) ein erster Arretiermechanismus (74) ausgebildet ist und wobei am proximalen Ende des genannten Gehäuses (8) ein zweiter Arretiermechanismus (76) ausgebildet ist, wobei der genannte erste und zweite Arretiermechanismus (74, 76) zusammenwirken, um das genannte Gehäuse (8) nach dem Drehen in eine Position, in der es im Wesentlichen auf die genannte Nadelnabe (4) ausgerichtet ist, fest an dem genannten Bund (6) zu halten, um die genannte Nadel (22) zu bedecken.

20. Verfahren zur Herstellung einer Nadelbaugruppe, das die folgenden Schritte beinhaltet:
a) Bereitstellen einer Nadelnabe (4) mit einem proximalen Abschnitt (18) und einem distalen Abschnitt (20) ;
b) festes Anbringen einer Nadel (22) an einem distalen Ende der genannten Nadelnabe (4);
c) schwenkbares Verbinden eines Gehäuses (8) mit einem Bund (6);
d) drehbares Montieren des genannten Bunds (6) um den distalen Abschnitt (20) der genannten Nadelnabe (4), und **gekennzeichnet durch**
e) Anbringen einer Nadel-(22)-Schutzkappe (12) an dem genannten Bund (6), so dass die Nadelschutzkappe nicht mit der Nadelnabe (4) in Kontakt kommt, um die sich vom distalen Ende der genannten Nadelnabe erstreckende Nadel (22) zu bedecken.

21. Verfahren nach Anspruch 20, das ferner die folgenden Schritte beinhaltet:
Entfernen der genannten Nadelschutzkappe (12) von dem genannten Bund (6) vor dem Gebrauch der genannten Nadel (22); und
Drehen des genannten Gehäuses (8) in eine Position, in der es im Wesentlichen entlang einer Längsachse der genannten Nadelnabe (4) ausgerichtet ist, um die genannte Nadel (22) zu bedecken.

22. Verfahren nach Anspruch 20, das ferner die folgenden Schritte beinhaltet:
Ausbilden eines ersten Eingriffsmechanismus (90) an der genannten Nadelschutzkappe (12) in der Nähe ihres offenen Endes (94);
Ausbilden eines zweiten Eingriffsmechanismus (68) an einem distalen Abschnitt (20) des genannten Bunds (6); und
wobei der genannte Schritt e die folgenden Schritte beinhaltet:
Positionieren der genannten Nadelschutzkappe (12) über der genannten Nadel (22); und
Zusammenstecken der genannten Nadelschutzkappe (12) mit dem genannten Bund (6), bis der genannte erste Eingriffsmechanismus (90) in den genannten zweiten Eingriffsmechanismus (68) eingreift.

23. Verfahren nach Anspruch 20, das ferner die folgenden Schritte beinhaltet:
Ausbilden einer Umfangsnut (90) in der Nähe eines offenen Endes (94) der genannten Nadelschutzkappe (12);
Ausbilden einer Rippe (68) umfangsmäßig an der Innenwand eines distalen Abschnitts (20) des genannten Bunds (6); und
wobei der genannte Schritt e die folgenden Schritte beinhaltet:
Positionieren der genannten Nadelschutzkappe (12) über der genannten Nadel (22); und
Ineingriffbringen der genannten Nadelschutzkappe (12) mit dem genannten Bund (6), bis die genannte Rippe des genannten Bunds (6) in der genannten Nut der genannten Nadelschutzkappe (12) steckt.

24. Verfahren nach Anspruch 20, wobei der genannte Schritt a die folgenden Schritte beinhaltet:
Ausbilden eines Luer-Endes (40) am proximalen Abschnitt (18) der genannten Nadelnabe (4); und
Ausbilden eines Rings (28) in beabstandeter Beziehung zum Umgeben des genannten Luer-Endes (40), wobei der genannte Ring (28) über eine distale Endwand (30) einstückig mit der genannten Nadelnabe (4) ausgebildet ist;
wobei ein Benutzer den genannten Ring leicht ergreifen kann, um die genannte Sicherheitsvorrichtung mit einer medizinischen Vorrichtung durch Schrauben des genannten Luer-Endes (40) der genannten Nadelnabe (4) auf ein Luer-Gegenende an der genannten medizinischen Vorrichtung zu koppeln.

25. Verfahren nach Anspruch 24, wobei der genannte Schritt des Ausbildens eines Rings (28) ferner den folgenden Schritt beinhaltet:
Ausbilden von wenigstens einem Fenster (42) an dem genannten Ring (28), damit der Benutzer das genannte Luer-Ende (40) der genannten Nadelnabe (4) und die genannte Nadelnabe (4) sehen kann.

26. Verfahren nach Anspruch 20, wobei der genannte Schritt a die folgenden Schritte beinhaltet:
Bereitstellen von wenigstens einem Flansch (44), der sich von dem genannten distalen Abschnitt (20) der genannten Nadelnabe (4) erstreckt; und
Positionieren des genannten Flansches (44) in einem vorbestimmten Abstand von einer Wand (30), die orthogonal von der genannten Nadelnabe (4) vorsteht, um einen Raum (52) zwischen dem genannten Flansch (44) und der genannten Wand umfangsmäßig um die genannte Nadelnabe (4) zu definieren;
wobei das Verfahren ferner die folgenden Schritte beinhaltet:
Ausbilden von wenigstens einem Vorsprung (62) an der Innenwand des genannten Bunds (6);
Dimensionieren des genannten Vorsprungs (62) so, dass er zu dem genannten, zwischen dem genannten Flansch (44) und der genannten Wand definierten Raum passt; und
Stecken des genannten Bunds (6) auf die genannte Nadelnabe (4), wobei der genannte Bund (6) nach dem Stecken in den genannten Raum um die genannte Nadelnabe (4) herum drehbar ist.

27. Verfahren nach Anspruch 20, das ferner den folgenden Schritt beinhaltet:
Bereitstellen einer Längsöffnung (80) entlang dem genannten Gehäuse (8) durch Ausbilden einer ersten und einer zweiten Lippe (82, 84), die jeweils im Wesentlichen entlang der Länge des genannten Gehäuses (8) verlaufen, wobei die genannte erste Lippe (82) einen Abschnitt der genannten zweiten Lippe (24) überlappt, wobei die genannte Öffnung (80) von der genannten Längsachse exzentrisch ist, wobei jede der genannten Lippen (82, 84) in Richtung auf das Innere des genannten Gehäuses (8) abgewinkelt ist, wobei sich die jeweiligen Winkel der genannten Lippen (82, 84) über die Länge des genannten Gehäuses (8) ändern, um eine Führung für die genannte Nadel (22) zu erzielen, so dass diese in einem Winkel durch die genannte Öffnung (80) leichtgängig in das genannte Gehäuse (8) eintreten kann, wenn das genannte Gehäuse (8) gedreht wird, um die genannte Nadel (22) zu bedecken, wobei die genannte Nadel (22) nach dem völligen Eintreten in das genannte Gehäuse (8) nicht von dem genannten Gehäuse (8) abgenommen werden kann.

28. Verfahren nach Anspruch 20, das ferner die folgenden Schritte beinhaltet:
Ausbilden eines ersten Arretiermechanismus (74) auf der Außenfläche des genannten Bunds (6); und
Ausbilden eines zweiten Arretiermechanismus (76) an einem proximalen Ende des genannten Gehäuses (8);
wobei der genannte erste und zweite Arretiermechanismus (74, 76) zusammenwirken, um das genannte Gehäuse (8) nach dem Drehen in eine Position, in der es im Wesentlichen mit der genannten Nadelnabe (4) ausgerichtet ist, fest an dem genannten Bund (6) zu halten, um die genannte Nadel (22) zu bedecken.

## Revendications

1. Appareil de sécurité, comprenant :
une embase d'aiguille (4) possédant une portion proximale (18) et une portion distale (20), une aiguille (22) s'étendant à partir d'une extrémité distale de ladite embase d'aiguille (4) ;
un collier (6) monté de façon rotative autour de la portion distale (20) de ladite embase d'aiguille (4) ;
un logement (8) raccordé de façon pivotante audit collier (6) ; et
un collier (6) de gaine d'aiguille (12) pour couvrir ladite aiguille (22) s'étendant à partir de l'extrémité distale de ladite embase d'aiguille (4), **caractérisé en ce que** la gaine d'aiguille (12) est attachée au collier (6) de sorte que la gaine d'aiguille n'entre pas en contact avec l'embase d'aiguille (4).

2. Appareil de sécurité selon la revendication 1, après que ladite gaine d'aiguille a été enlevée dudit collier (6), ledit logement (8) étant pivotable vers une position qui est sensiblement en alignement le long d'un axe longitudinal de ladite embase d'aiguille (4) pour couvrir ladite aiguille (22).

3. Appareil de sécurité selon la revendication 1, ladite gaine d'aiguille (12) possédant un premier mécanisme de solidarisation (90) à proximité de son extrémité ouverte (94), et ledit collier (6) possédant un deuxième mécanisme de solidarisation (68) lequel est formé au niveau de sa portion distale ; et
cas dans lequel lorsque ladite gaine d'aiguille (12) est positionnée au-dessus de ladite aiguille (22) et est accouplée avec ledit collier (6), ledit premier mécanisme de solidarisation (90) se solidarise avec ledit deuxième mécanisme de solidarisation (68) afin d'attacher ladite gaine d'aiguille (12) audit collier (6).

4. Appareil de sécurité selon la revendication 1, ladite gaine d'aiguille (12) possédant une rainure (90) formée de façon circonférentielle autour de celle-ci à proximité de son extrémité ouverte (94), et ledit collier (6) possédant une nervure (68) formée de façon circonférentielle au niveau de la paroi interne de son extrémité distale ; et
cas dans lequel ladite gaine d'aiguille (12) est attachée audit collier (6) lorsque ladite nervure dudit collier (6) s'accouple avec ladite rainure après que ladite gaine d'aiguille (12) est positionnée au-dessus de ladite aiguille (22) et se solidarise avec ledit collier (6).

5. Appareil de sécurité selon la revendication 1, ladite embase d'aiguille (4) comprenant un embout Luer (40) au niveau de sa portion proximale (18), une bague (28) entourant ledit embout Luer et qui est espacée de ce dernier, ladite bague faisant partie intégrante de ladite embase d'aiguille (4) par l'intermédiaire d'une paroi d'extrémité distale (30) s'étendant dans le plan transversal à partir de celle-ci ; et
cas dans lequel un utilisateur peut facilement agripper ladite bague pour coupler ledit dispositif de sécurité à un dispositif médical grâce à un accouplement par filetage dudit embout Luer de ladite embase d'aiguille (4) avec un embout Luer homologue au niveau dudit dispositif médical.

6. Appareil de sécurité selon la revendication 5, ladite bague comprenant au moins une fenêtre (42) pour permettre à l'utilisateur de visualiser ledit embout Luer de ladite embase d'aiguille (4) et ladite embase d'aiguille (4).

7. Appareil de sécurité selon la revendication 1, ladite embase d'aiguille (4) comprenant au moins une bride (44) laquelle s'étend depuis sa portion distale, ladite bride (44) étant positionnée à une distance prédéterminée par rapport à une paroi (30) faisant saillie dans le plan orthogonal à partir de ladite embase d'aiguille, un espace (52) étant formé entre ladite bride (44) et ladite paroi dans le plan circonférentiel autour de ladite embase d'aiguille (4) ; et
cas dans lequel ledit collier (6) comporte au moins une saillie (62) au niveau de la paroi interne de sa portion proximale (18), ladite saillie (62) étant dimensionnée pour s'adapter audit espace défini entre ladite bride (44) et ladite paroi lorsque ledit collier (6) est accouplé à ladite embase d'aiguille (4), ledit collier (6) étant apte à tourner autour de ladite embase d'aiguille (4) après avoir été adapté par accouplement audit espace.

8. Appareil de sécurité selon la revendication 1, ledit logement (8) comprenant une ouverture longitudinale (80) formée par des première et deuxième lèvres (82, 84), alors que chacune s'étend sensiblement le long de la longueur dudit logement (8), ladite première lèvre (82) chevauchant une portion de ladite deuxième lèvre (84) tandis que ladite ouverture (80) est décentrée par rapport audit axe longitudinal, chacune desdites lèvres (82, 84) étant inclinée vers le volume interne dudit logement (8), les angles respectifs desdites lèvres (82, 84) étant variés le long de la longueur dudit logement (8) pour servir de guide à ladite aiguille (22) pour lui permettre d'entrer sans à-coups dans ledit logement (8) suivant un certain angle à travers ladite ouverture (80) lorsque ledit logement (8) est pivoté afin de couvrir ladite aiguille (22), ladite aiguille (22) ne pouvant pas être enlevée dudit logement une fois que ladite aiguille (22) pénètre entièrement dans ledit logement (8).

9. Appareil de sécurité selon la revendication 1, ledit collier (6) ayant formé un mécanisme de verrouillage (74) au niveau de sa surface externe, et ledit logement (8) ayant formé un autre mécanisme de verrouillage (76) au niveau de son extrémité proximale, lesdits un et autre mécanismes de verrouillage (76) agissant mutuellement pour retenir de façon fixe ledit logement sur ledit collier (6) une fois que ledit logement (8) est pivoté vers une position qui est en alignement sensible avec ladite embase d'aiguille (4) afin de couvrir ladite aiguille (22).

10. Appareil de sécurité selon la revendication 9, ledit un mécanisme de verrouillage comprenant au moins un élément d'enclenchement unidirectionnel (74) lequel s'étend à partir de la surface externe dudit collier (6) ou dudit logement (8), et ledit autre mécanisme de verrouillage (76) comprenant au moins un orifice correspondant (76) au niveau dudit logement ou dudit collier (6), ledit un élément d'enclenchement unidirectionnel (74) étant couplé par accouplement audit orifice (76) pour retenir de façon fixe ledit logement (8) sur ledit collier (6) lorsque ledit logement (8) est pivoté pour couvrir ladite aiguille (22).

11. En combinaison, une embase d'aiguille (4) possédant une portion proximale (18) et une portion distale (20), ladite portion proximale (18) possédant un raccord Luer (40) et une bague (28) laquelle entoure ledit raccord Luer (40), mais est en relation d'espacement avec ce dernier, ladite portion distale (20) de ladite embase d'aiguille (4) possédant une extrémité distale à partir de laquelle s'étend une aiguille (22), un collier (6) possédant un logement (8) raccordé de façon pivotante à celui-ci, monté sur ladite portion distale (20) de ladite embase d'aiguille (4) et apte à tourner autour de cette portion distale.

12. Combinaison selon la revendication 11, comprenant en outre une gaine d'aiguille (12) raccordée audit collier (6), ladite gaine d'aiguille (12) pouvant être enlevée dudit collier (6) afin d'exposer ladite aiguille (22) en vue d'une utilisation.

13. Combinaison selon la revendication 12, ladite gaine d'aiguille (12) comprenant un premier mécanisme de solidarisation (90) à proximité de son extrémité ouverte (94), et ledit collier (6) comprenant un deuxième mécanisme de solidarisation (68) au niveau de sa portion distale, ledit premier mécanisme de solidarisation (90) se solidarisant avec ledit deuxième mécanisme de solidarisation (68) afin d'attacher ladite gaine d'aiguille (12) audit collier (6), lorsque ladite gaine d'aiguille (12) est positionnée au-dessus de ladite aiguille et est accouplée avec ledit collier (6).

14. Combinaison selon la revendication 12, ladite gaine d'aiguille (12) comprenant une rainure circonférentielle (90) à proximité de son extrémité ouverte (94), et ledit collier (6) comprenant une nervure circonférentielle (68) au niveau de sa portion distale, ladite nervure s'accouplant à ladite rainure lorsque ladite gaine d'aiguille (12) est positionnée au-dessus de ladite aiguille (12) et est montée sur ledit collier (6).

15. Combinaison selon la revendication 11, ladite bague (28) comprenant au moins une fenêtre (42) sur sa paroi latérale (32) pour permettre une visualisation dudit raccord Luer et de ladite portion proximale (18) de ladite embase d'aiguille (4).

16. Combinaison selon la revendication 11, ladite bague étant adaptable pour être utilisée par un utilisateur pour lui permettre d'agripper ladite embase d'aiguille (4) afin de raccorder ledit raccord Luer (40) à un raccord Luer correspondant d'un dispositif médical.

17. Combinaison selon la revendication 11, ladite embase d'aiguille (4) comprenant une pluralité de brides (44) lesquelles s'étendent depuis sa portion distale (20), lesdites brides (44) étant positionnées à une distance prédéterminée par rapport à une paroi (30) faisant saillie dans le plan orthogonal à partir de ladite embase d'aiguille (4), un espace (52) étant défini entre lesdites brides (44) et ladite paroi dans le plan circonférentiel autour de ladite embase d'aiguille (4), et ledit collier (6) comprenant une pluralité de saillies (62) au niveau de la paroi interne de sa portion proximale (18), lesdites saillies (62) étant dimensionnées de façon à être adaptées audit espace lorsque ledit collier (6) est accouplé à ladite embase d'aiguille (4), ledit collier (6) étant apte à tourner autour de ladite embase d'aiguille (4) après avoir été adapté par accouplement audit espace.

18. Combinaison selon la revendication 11, ledit logement (8) comprenant une ouverture longitudinale (80) formée par des première et deuxième lèvres (82, 84), alors que chacune s'étend sensiblement le long de la longueur dudit logement, ladite première lèvre (82) chevauchant une portion de ladite deuxième lèvre (84) tandis que ladite ouverture est décentrée par rapport audit axe longitudinal, chacune desdites lèvres (82, 84) étant inclinée vers le volume interne dudit logement (8), les angles respectifs desdites lèvres (82, 84) étant variés le long de la longueur dudit logement (8) pour servir de guide à ladite aiguille (22) pour lui permettre d'entrer sans à-coups dans ledit logement (8) suivant un certain angle à travers ladite ouverture (80) lorsque ledit logement (8) est pivoté afin de couvrir ladite aiguille (22), ladite aiguille (22) ne pouvant pas être enlevée dudit logement (8) une fois que ladite aiguille (22) pénètre entièrement dans ledit logement (8).

19. Combinaison selon la revendication 11, ledit collier (6) ayant formé un premier mécanisme de verrouillage (74) au niveau de sa surface externe, et ledit logement (8) ayant formé un deuxième mécanisme de verrouillage (76) au niveau de son extrémité proximale, lesdits premier et deuxième mécanismes de verrouillage (74, 76) agissant mutuellement pour retenir de façon fixe ledit logement sur ledit collier (6) une fois que ledit logement (8) est pivoté vers une position qui est en alignement sensible avec ladite embase d'aiguille (4) afin de couvrir ladite aiguille (22).

20. Procédé de réalisation d'un ensemble aiguille, comprenant les étapes consistant à :
a) procurer une embase d'aiguille (4) possédant une portion proximale (18) et une portion distale (20) ;
b) attacher de façon fixe une aiguille (22) à une extrémité distale de ladite embase d'aiguille (4) ;
c) raccorder de façon pivotante un logement (8) à un collier (6) ;
d) monter de façon rotative ledit collier (6) autour de la portion distale (20) de ladite embase d'aiguille (4); et **caractérisé par** l'opération consistant à
e) attacher une gaine (12) d'aiguille (22) audit collier (6) de sorte que la gaine d'aiguille n'entre pas en contact avec l'embase d'aiguille (4) pour couvrir ladite aiguille (22) s'étendant à partir de l'extrémité distale de ladite embase d'aiguille.

21. Procédé selon la revendication 20, comprenant en outre les étapes consistant à :
enlever ladite gaine d'aiguille (12) dudit collier (6) avant d'utiliser ladite aiguille (22) ; et
faire pivoter ledit logement (8) vers une position qui est sensiblement en alignement le long d'un axe longitudinal de ladite embase d'aiguille (4) afin de couvrir ladite aiguille (22).

22. Procédé selon la revendication 20, comprenant en outre les étapes consistant à :
former un premier mécanisme de solidarisation (90) sur ladite gaine d'aiguille (12) à proximité de son extrémité ouverte (94) ;
former un deuxième mécanisme de solidarisation (68) sur une portion distale (20) dudit collier (6) ; et
cas dans lequel ladite étape « e » comprend les étapes consistant à :
positionner ladite gaine d'aiguille (12) au-dessus de ladite aiguille (22) ; et
accoupler ladite gaine d'aiguille (12) avec ledit collier (6) jusqu'à ce que ledit premier mécanisme de solidarisation (90) se solidarise avec ledit deuxième mécanisme de solidarisation (68).

23. Procédé selon la revendication 20, comprenant en outre les étapes consistant à :
former une rainure circonférentielle (90) à proximité d'une extrémité ouverte (94) de ladite gaine d'aiguille (12) ;
former une nervure (68) de façon circonférentielle au niveau de la paroi interne d'une portion distale (20) dudit collier (6) ; et
cas dans lequel ladite étape « e » comprend les étapes consistant à :
positionner ladite gaine d'aiguille (12) au-dessus de ladite aiguille (22) ; et
solidariser ladite gaine d'aiguille (12) audit collier (6) jusqu'à ce que ladite nervure dudit collier (6) s'accouple avec ladite rainure de ladite gaine d'aiguille (12).

24. Procédé selon la revendication 20, ladite étape « a » comprenant les étapes consistant à :
former un embout Luer (40) au niveau de la portion proximale (18) de ladite embase d'aiguille (4) ; et
former une bague (28), en relation d'espacement, afin d'entourer ledit embout Luer (40), ladite bague (28) faisant partie intégrante de ladite embase d'aiguille (4) par l'intermédiaire d'une paroi d'extrémité distale (30) ;
cas dans lequel un utilisateur peut facilement agripper ladite bague pour coupler ledit dispositif de sécurité à un dispositif médical grâce à un accouplement par filetage dudit embout Luer (40) de ladite embase d'aiguille (4) avec un embout Luer homologue au niveau dudit dispositif médical.

25. Procédé selon la revendication 24, ladite étape de formation d'une bague (28) comprenant en outre l'étape consistant à :
former au moins une fenêtre (42) sur ladite bague (28) pour permettre à l'utilisateur de visualiser ledit embout Luer (40) de ladite embase d'aiguille (4) et ladite embase d'aiguille (4).

26. Procédé selon la revendication 20, ladite étape « a » comprenant les étapes consistant à :
procurer au moins une bride (44) laquelle s'étend depuis ladite portion distale (20) de ladite embase d'aiguille (4) ; et
positionner ladite bride (44) à une distance prédéterminée par rapport à une paroi (30) faisant saillie dans le plan orthogonal à partir de ladite embase d'aiguille (4) afin de définir un espace (52) entre ladite bride (44) et ladite paroi dans le plan circonférentiel autour de ladite embase d'aiguille (4) ;
cas dans lequel le procédé comprend en outre les étapes consistant à :
former au moins une saillie (62) au niveau de la paroi interne dudit collier (6) ;
dimensionner ladite saillie (62) de façon à l'adapter audit espace défini entre ladite bride (44) et ladite paroi ; et
accoupler ledit collier (6) à ladite embase d'aiguille (4), ledit collier (6) étant apte à tourner autour de ladite embase d'aiguille (4) après avoir été accouplé audit espace.

27. Procédé selon la revendication 20, comprenant en outre l'étape consistant à :
procurer une ouverture longitudinale (80) le long dudit logement (8) en formant des première et deuxième lèvres (82, 84), alors que chacune s'étend sensiblement le long de la longueur dudit logement (8), ladite première lèvre (82) chevauchant une portion de ladite deuxième lèvre (24) tandis que ladite ouverture (80) est décentrée par rapport audit axe longitudinal, chacune desdites lèvres (82, 84) étant inclinée vers le volume interne dudit logement (8), les angles respectifs desdites lèvres (82, 84) étant variés le long de la longueur dudit logement (8) pour servir de guide à ladite aiguille (22) pour lui permettre d'entrer sans à-coups dans ledit logement (8) suivant un certain angle à travers ladite ouverture (80) lorsque ledit logement (8) est pivoté afin de couvrir ladite aiguille (22), ladite aiguille (22) ne pouvant pas être enlevée dudit logement (8) une fois que ladite aiguille (22) pénètre entièrement dans ledit logement (8).

28. Procédé selon la revendication 20, comprenant en outre les étapes consistant à :
former un premier mécanisme de verrouillage (74) au niveau de la surface externe dudit collier (6) ; et
former un deuxième mécanisme de verrouillage (76) au niveau d'une extrémité proximale dudit logement (8) ;
cas dans lequel lesdits premier et deuxième mécanismes de verrouillage (74, 76) agissent mutuellement pour retenir de façon fixe ledit logement (8) sur ledit collier (6) une fois que ledit logement (8) est pivoté vers une position qui est en alignement sensible avec ladite embase d'aiguille (4) afin de couvrir ladite aiguille (22).
